# EUROPEAN PATENT APPLICATION

(11) **EP 2 810 695 A1**
(43) Date of publication of application: **10.12.2014**
(21) Application number: 13170932.1
(22) Date of filing: 06.06.2013
(51) Int. Cl.: A61P 31/04, A61K 38/17, A61K 38/44, A61K 38/57, A61K 9/00, A61P 15/14

(54) **Compositions and Method for controlling infections in non-human mammals using acute phase proteins**

(71) Applicant: Ceva Sante Animale, 33500 Libourne Cedex (FR)
(72) Inventor: Bach Ariza, Alejandro, 08173 SAN CUGAT (ES); Pares, Sylvia, 08211 CASTELLAR DEL VALLES (ES); Domenech, Anna, 08201 SABADELL (ES); Aris, Anna, 08173 ST CUGAT DEL VALLES (ES)
(74) Representative: Becker, Philippe

(57) **Abstract**

The present invention relates to compositions and methods for controlling infections in non-human mammals, particularly intra-mammary infections (IMIs). The invention is particularly suited to control infections and enhance the welfare of non-human mammals, preferably bovine, by improving mammary gland involution during dry off period. The invention is based on the use of acute phase proteins, more preferably Serum Amyloid A (SAA), even more preferably the isoform 3 (SAA-3).

## Description

The present invention relates to compositions and methods for controlling infections in non-human mammals, particularly intra-mammary infections (IMIs). The invention is particularly suited to control infections and enhance the welfare of non-human mammals, preferably bovine, by improving mammary gland involution during dry off period. The invention is based on the use of acute phase proteins, more preferably Serum Amyloid A (SAA), even more preferably the isoform 3 (SAA-3).

### Introduction

Milk production is maximized when non-human mammals are pregnant for 70% of the time for each lactation. Between lactations a dry period is necessary to renew senescent epithelial cells of the mammary gland and ensure optimal production of milk in the next lactation. Because of the hormonal levels resulting from pregnancy status in non-human mammals and especially in ruminants such as bovine, involution of the mammary gland is slower than in other species where involution does not coexists with pregnancy. This requires long dry periods of 60 days, which significantly reduce the duration of the productive period. The dry period is characterized by two main phases of cell turnover: the mammary gland involution, characterized by acute apoptosis, and cell regeneration before the next lactation. A peak of apoptosis is observed during the first 72 hours post milk cessation, continuing along the rest of dry period and combined by increasing cell proliferation. Physiologically, milk stasis produced by removing the milking of the non-human mammal, along with protein and hormonal factors such as lactogenic hormones drop, induces active gland involution and in turn stimulates contraction of the parenchyma and stops milk secretion.

Mammary gland tissue is composed by epithelial secretory cells and stromal tissue with different cell types such as fibroblast and immune cells. Both tissues are separated by basement membrane, a type of extracellular matrix (ECM), which influences the development and biology of the mammary gland. The ECM directly establishes and maintains the mammary gland differentiated state and prevents apoptosis. The proteolytic destruction of the basement membrane induces apoptosis and involution of mammary gland. There are several proteases involved in the extracellular matrix degradation but basically the matrix metalloproteinases (MMPs) are key enzymes in this process. Moreover, other functions have been assigned to MMPs such as release of growth factors and cytokines, which in turn modulate other key aspects of dry period such as the immune system activation and cell growth.

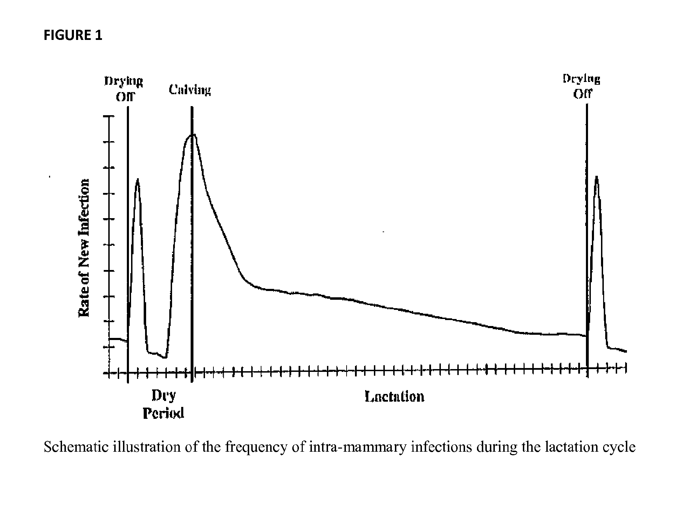

The mammary gland in said non-human mammals experience dramatic changes in susceptibility to infections during the lactation cycle, as illustrated schematically in Figure 1, and over 60% of new intra-mammary infections (IMIs) are observed in the early dry period. Indeed, milk stasis increases pressure within mammary gland promoting milk leakage and susceptibility to bacterial infection. Furthermore, milk is a rich media for bacterial growth. In addition, animals with high milk yields are more vulnerable to contract intra-mammary infections since they have a delayed formation of the keratin plug and increased time of teat canal occlusion. The IMIs contracted during the dry period have been associated with increase infections prevalence in the next lactation, which cause loss of milk production and decreased milk quality

The early dry period offers therefore an ideal opportunity to manage, through judicious use of treatments, the IMIs. Indeed, to avoid the high risk of IMIs along this period and during the subsequent lactation, antibiotics are infused into the mammary gland routinely. However, this practice is associated to the risk to develop resistances to antimicrobials that are identical in humans and animals. Stimulation of non-human mammal immunity at the beginning of the drying period may be relevant not only to improve the efficacy of antibiotics in cases of infection with bacteria escaping spectrum antibiotics, but also could eventually replace the use of antibiotics as a preventive agent in the drying period.

### Summary of the Invention

The present invention provides novel compositions and methods for controlling intra-mammary infections (IMIs) in non-human mammals. The invention is more particularly based on the use of acute phase proteins, more preferably Serum Amyloid A (SAA), even more preferably the isoform 3 (SAA-3), to control IMI in non-human mammals.

An object of the invention therefore resides in an acute phase protein, preferably a SAA-3 protein, or a composition comprising an acute phase protein, preferably a SAA-3 protein, for use to control IMI in a non-human mammal. A further object of the invention is a method for controlling IMI in a non-human mammal, comprising administering to the non-human mammal an effective amount of an acute phase protein, preferably a SAA-3 protein. A further object of the invention resides in the use of an acute phase protein for controlling IMI in a non-human mammal. The compositions, methods or uses of the invention may be used alone (i.e., in replacement of current treatments) or in combination with current treatments (e.g., antibiotics, antioxidant, antiprolactin, antigens, or teat sealants), to improve their efficacy. Also, the invention may use one acute phase protein (for instance a SAA-3 protein) or several distinct acute phase proteins, in combination.

More particularly, the invention provides acute phase protein-based compositions and methods for controlling IMIs in non-human mammals by improving the efficacy of conventional intra-mammary therapies, preferably antibiotics, antiprolactin, and /or teat sealants.

Even more particularly, the invention provides SAA-3-based compositions and methods for controlling IMIs in non-human mammals by replacing the use of antibiotics.

The invention is particularly adapted and efficient for treating the non-human mammals during the dry period, thereby controlling IMI during the dry period and/or in the subsequent lactation period(s). A particular object of the invention relates to novel acute phase protein-based compositions and methods to control IMIs during the dry period and/or in the subsequent lactation. Preferably, the acute phase protein composition is administered during the dry period, more preferably at the beginning of the dry period.

A further more particular object of the invention relates to novel compositions and methods comprising an acute phase protein, preferably a SAA-3 protein for use to control mastitis.

Another object of the invention relates to novel compositions and methods comprising an acute phase protein, more preferably Serum Amyloid A (SAA), even more preferably the isoform 3 (SAA-3), for enhancing the welfare of non-human mammals by improving mammary gland involution.

More particularly, the invention provides acute phase protein-based compositions and methods for enhancing udder health by improving mammary gland involution.

Even more particularly, the invention provides acute phase protein-based compositions and methods for enhancing udder inflammation by improving mammary gland involution.

The acute phase proteins protein for use in the present invention are preferably in pure form or in association with one or several pharmaceutically acceptable excipients or carriers.

A further object of the invention relates to a method for administering (together or separately, simultaneously or sequentially) to a non-human mammal a composition comprising an acute phase protein, either alone or in combination with other acute phase protein, antibiotics, antioxydants, teat seleants, antigens or an antiprolactin compound, preferably an agonist of dopamine receptors, more preferably an ergoline-derived dopamine and even more preferably the cabergoline. The invention also relates to a combination treatment for bovine comprising a SAA-3 protein and an antiprolactin compound.

The invention may be used in any non-human mammals, preferably in any ungulate or ruminant, such as bovine, ovine, equine, sheep, or goats.

The compositions and method of the invention are particularly effective for controlling intra-mammary infections (IMIs), improving udder welfare and enhancing natural immunity system in non-human mammals. This invention is particularly effective for protecting against or for preventing intra-mammary infection *by S. aureus.* The invention is more particularly effective during dry period, particularly during early stage of dry period. The invention is particularly effective to control IMIs and/or co-infection or enhance welfare by administering SAA-3 alone or in combination with a conventional treatment such as for example antibiotics or teat sealants.

### Legend to the Figures

**Fig 1****:** Schematic illustration of the frequency of intra-mammary infections during the lactation cycle.

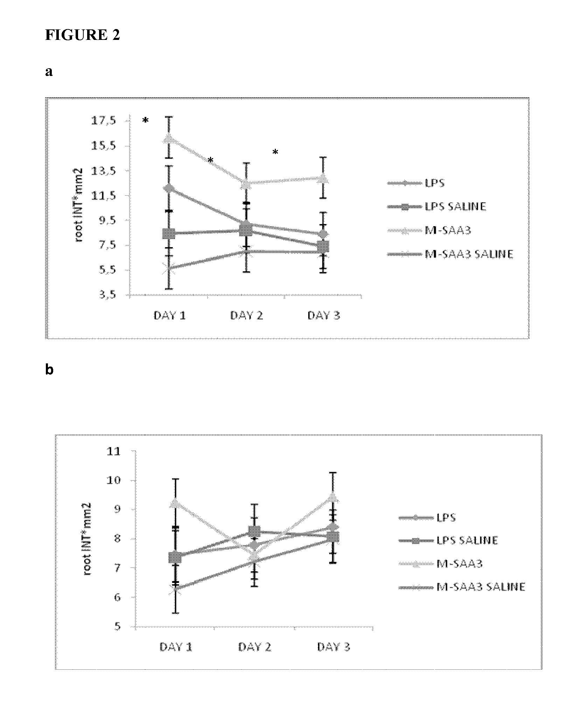

**Fig 2****:** Metalloproteinase (MMP) activity during the early dry period. Metalloproteinase activity is represented by the root of INT*mm2 ± SEM. Data were obtained from band quantification of zymography gels using the Quantity One software during the 3 days post dry off, for MMP-9 (a) and MMP-2 (b).Treatments with asterisk differ (P < 0.05).

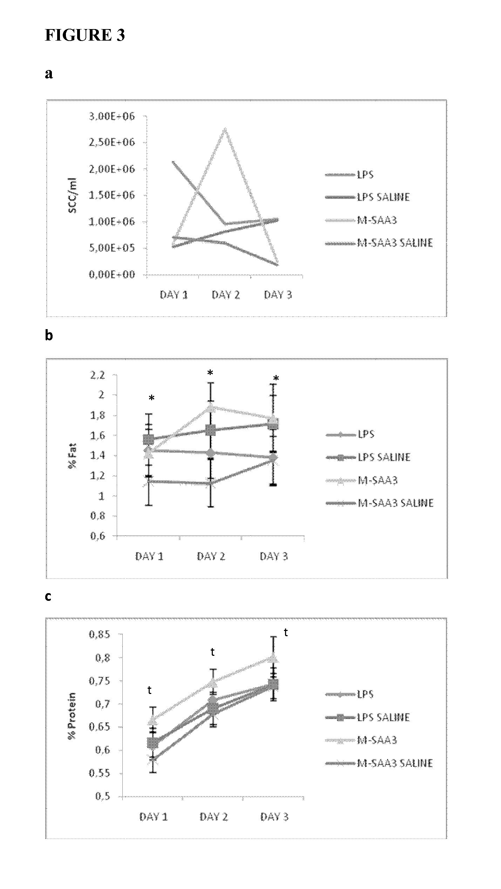

**Fig 3****:** Somatic Cell Count (SCC), fat and protein content during the early dry period. The SCC (a) are represented as SCC/ml ± SEM (transformed data). Fat (b) and protein (c) concentration (data represented as percentage ± SEM) during the 3 days post dry off). Treatments with asterisk differ (*P* < 0.05) and *t* (*P* < 0.1).

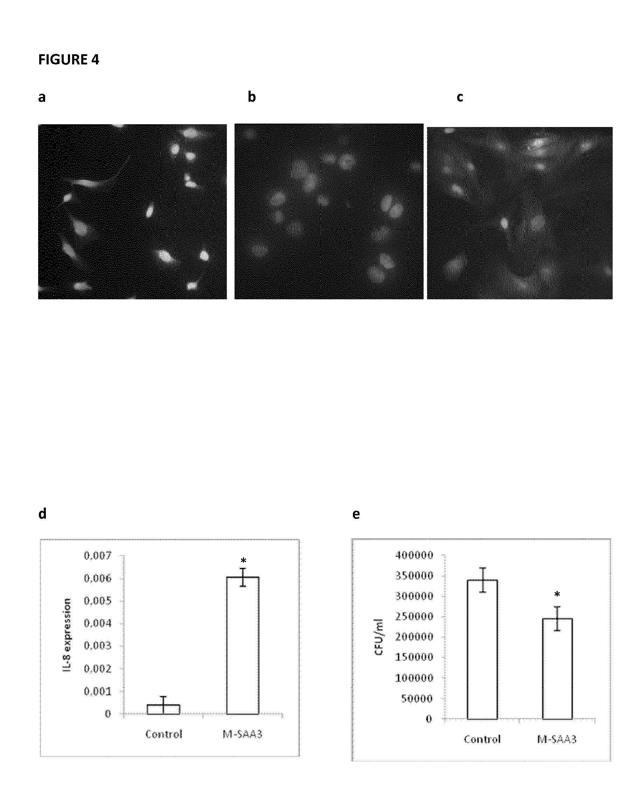

**Fig 4****:** Primary mammary gland cultures. Immunofluorescence of primary mammary cultures with pan-cytokeratin. HeLa cells (a) as positive control, THP-1 macrophages (b) as negative control and mammary cultures (c). Graphs represent the gene expression of IL-8 (a) and viable cell counts of internalized *S*. *aureus* (b). Bars represent the means ± SEM. Bars with asterisk are significantly different (*P* < 0.05).

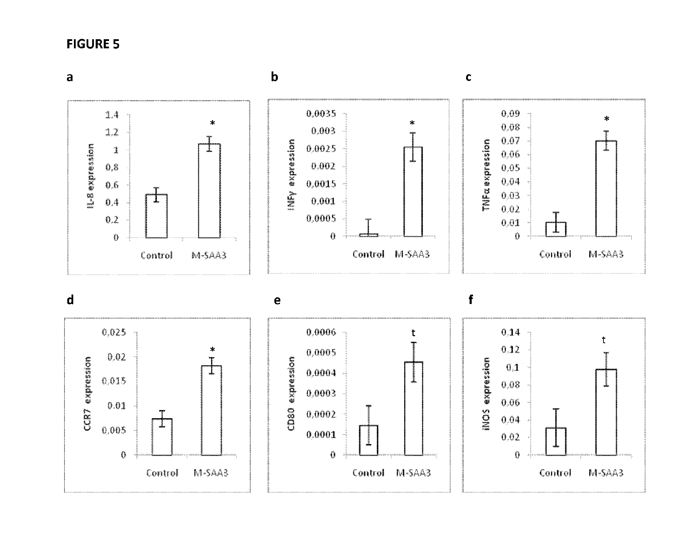

**Fig 5****:** Mammary SAA3 effect on gene expression in dendritic cells. Gene expression of IL-8 (a), INFγ (b), TNFα (c), CCR7 (d), CD80 (e) and iNOS (f). Bars represent the means ± SEM for the different treatments. Bars with asterisk are significantly different (*P* < 0.05) and *t* (*P* < 0.1).

### Detailed description of the invention

The present invention resides in the use of acute phase proteins, more preferably Serum Amyloid A (SAA), even more preferably the isoform 3 (SAA-3), as an active ingredient for controlling intra-mammary infections (IMIs) and/or for enhancing the welfare and/or boosting or stimulating natural immunity in non-human mammals, preferably in bovine.

The invention proposes, for the first time, the use of a such proteins, particularly SAA-3 protein, as an immune agent in non-human mammals, as well as to activate mammary gland involution.

The present invention surprisingly demonstrates that acute phase proteins, particularly SAA-3 protein, have the ability to increase the recruitment of immune cells (monocyte isolation, differentiation to dendritic cells and Interleukin 8 expression pattern), to stimulate mammary gland involution by enhancing metalloproteinase activity, modifying the Somatic Cell Counts as well as milk fat and protein content, and to inhibit bacterial translocation such as for example *Staphylococcus aureus* in mammary gland primary cultures.

### Acute Phase Proteins

Mammals respond to tissue injury, trauma or infection by executing a complex series of biological reactions in an effort to prevent further tissue damage, to initiate repair of damaged tissue, and to isolate and destroy infective organisms. This process is referred to as the inflammatory response. The early and intermediate stages of the inflammatory response are referred to as the acute phase response and involve a wide variety of protein mediators, including cytokines, interleukins and tumor necrosis factor which are reviewed by Steel & Whitehead (Immunology Today 15: 81-87, 1994).

The term "acute phase proteins" designates, within the context of this invention, a group of plasmatic proteins whose plasma concentration increases or decreases in response to tissue injury, acute infections, bums, or chronic inflammation in a non-human mammal. This group includes, more specifically, serum amyloid A (SAA) proteins, alpha-1 acid glycoprotein, alpha-1 antitrypsin, haptoglobins, fibrinogen, C-reactive protein, ferritin, ceruloplasmin and complement factors. A most preferred acute phase protein for use in the invention is an SAA protein.

Serum amyloid A (SAA) proteins are small acute phase proteins that accumulate and associate rapidly with high-density lipoprotein 3 (HDL3) during the acute phase of the inflammatory response. This family of acute phase proteins is produced in all vertebrates investigated to date and, depending on the species, three or four genetic loci that encode SAA have been identified. These SAA genes coding for SAA isoforms are differentially expressed hepatically and/or extra hepatically. Three major isoforms have been characterized, SAA-1, SAA-2, and SAA-3. SAA-3 is the most preferred SAA protein for use in the present invention.

An isolated and purified bovine colostrum associated SAA-3 protein has been described in US7,214,512. According to this patent, the SAA-3 protein and more particularly its TFLK highly conserved region present in the N- terminal region motif, has been shown to stimulate mucin production in the intestine, more specifically MUC3.

A bovine mammary SAA-3 isoform (M-SAA3) having 97% homology to colostrum associated SAA-3 protein has been purified from milk, which has been reported to have various activities in the animal such as chemotaxis of immune cells or cytokine modulation.

However, no effect of acute phase proteins such as SAA3 on the recruitment of immune cells (monocyte isolation, differentiation to dendritic cells and Interleukin 8 expression pattern) or on the mammary gland involution (e.g.; enhancing metalloproteinase activity, modifying the Somatic Cell Counts as well as milk fat and protein content) or on the bacterial translocation inhibition (such as for example *Staphylococcus aureus*) in mammary gland has ever been reported or suggested so far.

Within the context of this invention, the term "SAA-3"or "M-SAA3" designates a protein or a peptide comprising (i) the entire amino acid sequence of SEQ ID NO: 2, or (ii) a fragment of SEQ ID NO: 2 having at least 20 consecutive amino acids of SEQ ID NO: 2, preferably at least 25, 30, 35, 40 or at least 50 consecutive amino acids of SEQ ID NO: 2, or (iii) a sequence having at least 80% identity to SEQ ID NO: 2, preferably at least 85%, 90%, 95% or 97% sequence identity to SEQ ID NO: 2. The terms are used interchangeably and include but are not limited to the sequences disclosed herein, their conservatively modified variants, regardless of source and any other variants which retain the biological properties of the SAA-3 or M-SAA3.

Preferably, the term SAA-3 designates a protein or a peptide as defined above having the ability to stimulate Interleukin-8 (IL-8) production in mammary gland cells, or to increase somatic cell counts in mammary gland, or to reduce *in vitro* infection of mammary epithelial cells by S. *aureus* by at least 15%.

It is preferred to use an acute phase protein derived from a species to which the treatment is intended.

The term "sequence identity" as applied to nucleic acid or protein sequences, refers to the quantification (usually percentage) of nucleotide or amino acid residue matches between at least two sequences aligned using a standardized algorithm such as Smith-Waterman alignment (Smith and Waterman (1981) J Mol Biol 147:195-197), CLUSTALW (Thompson et al. (1994) Nucleic Acids Res 22:4673-4680), or BLAST2 (Altschul et al. (1997) Nucleic Acids Res 25:3389-3402). BLAST2 may be used in a standardized and reproducible way to insert gaps in one of the sequences in order to optimize alignment and to achieve a more meaningful comparison between them.

The acute phase protein may be an isolated protein of natural origin, a synthetic protein, or a recombinant protein.

The terms" proteins" or "peptide" are used interchangeably herein to refer to a polymer of amino acid residues. The terms apply to amino acid polymers in which one or more amino acid residue is an artificial chemical analogue of a corresponding naturally occurring amino acid, as well as to naturally occurring amino acid polymers. The essential nature of such analogues of naturally occurring amino acids is that, when incorporated into a protein, that protein is specifically reactive to antibodies elicited to the same protein but consisting entirely of naturally occurring amino acids.

The protein may be modified such as e.g., glycosylated, and/or may comprise modified and/or unnatural amino acids. The term "modified" is also inclusive of modifications including, but not limited to, phosphorylation, glycosylation, lipid attachment, sulfatation, gamma- carboxylation of glutamic acid residues, hydroxylation and ADP-ribosylation. It will be appreciated, as is well known and as noted above, that proteins are not entirely linear. For instance, proteins may be branched as a result of ubiquitination, and they may be circular, with or without branching, generally as a result of posttranslational events, including natural processing event and events brought about by human manipulation which do not occur naturally. Circular, branched and branched circular protein may be synthesized by non-translation natural process and by entirely synthetic methods, as well.

The term "recombinant" includes reference to a cell or vector, that has been modified by the introduction of a heterologous nucleic acid or that the cell is derived from a cell so modified. Thus, for example, recombinant cells express genes that are not found in identical form within the native (non-recombinant) form of the cell or express native genes that are otherwise abnormally expressed, under-expressed or not expressed at all as a result of deliberate human intervention. The term "recombinant" as used herein does not encompass the alteration of the cell or vector by naturally occurring events (e. g., spontaneous mutation, natural transformation/transduction/transposition) such as those occurring without deliberate human intervention. A recombinant protein or peptide is a protein or peptide obtainable from a recombinant cell.

The term "vector" includes a replicon, such as plasmid, phage, cosmid, or virus to which another nucleic acid segment may be operably inserted so as to bring about the replication or expression of the segment.

A protein or peptide for use in the invention (e.g., a SAA-3 protein or peptide) may be in isolated or purified form, preferably devoid of other proteins, preferably at least 90%, 95%, 96%, 97%, 98% or more pure.

### Mode of administration

Pharmaceutical compositions or proteins according to the present invention can be administered according to many ways of administration that are well known per se in the field and adapted to the treated non-human mammal and disease. They are preferably administered cutaneously, orally, parenterally or by infusion. They are in appropriate forms for the selected mode of administration. They can therefore be in the form of a solution or an oral or injectable suspension liquid, or in a solid or semi-solid form, in powder form, capsule, granule, sugar coated tablet, soft capsule, sprays, caplet, pills, tablets, or pastes.

Advantageously, the acute phase proteins, or the pharmaceutical compositions comprising an acute phase protein, are administered by intra-mammary infusion(s).

In a further preferred embodiment, the composition in the form of a viscous paste.

The compositions and methods of the invention usually contain or use an "effective amount" of an acute phase protein, preferably a SAA-3 protein, e.g., an amount of said protein sufficient to cause a statistic effect in vivo and/or in-vitro on the recruitment of immune cells (e.g., monocyte isolation, differentiation to dendritic cells and/or Interleukin 8 expression pattern), on the stimulation of mammary gland involution (by e.g., enhancing metalloproteinase activity, or modifying the Somatic Cell Counts, or increasing milk fat and/or protein content), or on the inhibition of bacterial translocation such as for example *Staphylococcus aureus* in mammary gland primary cultures.

Depending on the formulations and protein, the pharmaceutical compositions may further comprise conventional pharmaceutically acceptable ingredients for the preparation of liquid or solid formulations to be administered cutaneously, orally, parenterally or by infusion. Moreover, in the case of oral formulations, these can be administered directly to the non-human mammals or can be mixed into the food.

Furthermore, the compositions according to the invention may comprise depending on the type of formulation, a solvent, a flowing agent, a lubricant and any suitable mass excipient such as lactose, cellulose or starch. As lubricant, one can use stearic acid, magnesium stearate, L-leucine or for example, glyceryl tribehenate. As a disintegrating agent, one can use sodium carboxymethyl starch, or reticulated sodium carboxymethyl starch. As a flowing agent, one can use pure silicon or silicon dioxide colloidal. Oral solid forms can be in the form of tablets covered with coating.

Suitable compositions of the invention are prepared by mixing effective therapeutic quantities of at least one anti prolactinic compound as described previously with a solvent, a pH regulator, a buffer agent, a suspending agent, a solubilization agent, a stabilizer, an agent of tonicity and/or a preservative, and by transforming the mix with a traditional process for injection or infusion. Examples of solvents are oily solvents such as medium chain triglycerides in C8-C10, or a mix of capric acid, caprylic acid, and triglycerides, such as those marketed under the name of Myglio1812. Injectable preparations can be freeze-dried according to a traditional process.

Examples of suspending agents include methylcellulose, polysorbate 80, hydroxyethyl cellulose, xanthan gum, sodium carboxymethyl cellulose, sorbitane monolaureate polyethylene. Examples of solubilization agents include ricin oil solidified by polyoxyethylene, polysorbate 80, nicotinamide, sorbitane monolaureate polyethoxylated, macrogol and ester ethyl fatty ricin acid. Besides, the stabilizing agent includes sodium sulfate, sodium metasulfate and ester, while the preservative includes methyl p-hydroxybenzoate, ethyl p-hydroxybenzoate, sorbic acid, benzyl alcohol, phenol, cresol and chlorocresol. One example of the agent of tonicity is mannitol. During the preparation of solutions and injectable suspensions, it is better to ensure that they are isotonic with blood.

Advantageously, the pharmaceutical compositions of the present invention can be administered in association with standard treatments of IMIs. Examples of standard care or prophylactic compositions of IMIs are local disinfectants for udders, teat sealants, antibiotics such as penicillins of group M, cephalosporine, gentamycin or colistine or even enzymes such as lysozymes or muramidase, antiprolactin compound such as ergot-derived dopamine receptor agonist and more specially cabergoline.

### Controlling intra-mammary infections (IMIs)

The compositions and methods of the invention are particularly suited and efficient for controlling IMIs in non-human mammals. As shown in the experimental section, the compositions and methods effectively block or reduce bacterial infection in the mammary gland, both in vitro and in vivo. The present invention surprisingly demonstrates that SAA-3 protein has the ability to inhibit bacterial translocation such as for example *Staphylococcus aureus* in mammary gland.

The compositions and methods are particularly effective for treatment during or shortly before the dry period.

Exposure to environmental pathogens is likely to continue throughout the dry period. These organisms are primarily contracted from contamination of udders by manure and bedding. There are different rates of intra-mammary infections (IMIs) caused by the various environmental agents as the dry period progresses and the profile of these pathogens agents will vary between farms and in different parts of the world. However, generally it can be expected that Gram positive bacteria are contagious IMIs pathogens that play a more important role in non-human mammals especially bovine with higher bulk milk Somatic Cell Count (SCC), whilst in low bulk milk SCC, environmental pathogens and in particular Gram negative bacteria are more influential. For example, infections with environmental *Streptococci, Klebsiella,* and *Enterobacter* occur more frequently early in the dry period. On the other hand, *E. coli* infections tend to occur immediately before and after calving. Generally, the predominant pathogen organisms for bovine IMIs are *Staphylococcus aureus, Escherichia coli, Streptococcus uberis, Streptococcus dysgalactiae* and coagulase negative *staphylococcus spp.*

Intra-mammary infections present at calving can have arisen either as a result of persistence of infections from the previous lactation or as a result of new infections acquired during the dry period. The vast majority of infections present in the late dry period are newly acquired rather than persisting from drying off. Although persistence is generally rare, Gram positive bacteria are more likely to persist through the dry period than gran negative bacteria.

New infections during the dry period are extremely important and have been shown to occur at up to 10 times the rate of new infections during lactation. In low-bulk milk SCC herds (<250 000 cells/mL), *Escherichia Coli* and *Streptococcus uberis* are usually the most frequent causes of new infections and research suggests that over 60% of new infections are caused by these organisms. Approximately 60% of all clinical mastitis occurring in the first 100 days of lactation can be attributed to IMIs acquired during the dry period, clearly demonstrating the importance and influence of the dry period in subsequent lactation IMIs infection.

Undoubtedly, controlling the development of IMIs during the dry period is a key point to manage efficiently mastitis and other infections which occur during this period and in the subsequent lactation. Moreover, controlling IMIs participate also to the welfare of non-human mammals by decreasing for example mammary pain.

A particular object of the invention therefore resides in a composition or method for controlling IMI during the dry period in a non-human mammal using an acute phase protein, preferably a SAA protein, more preferably a SAA3 protein. Another particular object of the invention resides in a composition or method for controlling new bacterial infections during dry period using an acute phase protein, preferably a SAA protein, more preferably a SAA3 protein.

Within the context of this invention, the term "controlling" or "control" in relation to an infection refers to the reduction of the occurrence or severity of said infection or infectious disease, and/or to at least a reduction of the likelihood, the risk, the susceptibility, the severity, the progression or the consequence of an infection.

The control of IMI particularly designates the prevention of IMIs, e.g., any reduction of an infection or invasion or translocation by a pathogen (e.g., bacterium), preferably any reduction by at least 15%, 20%, or more.

Controlling an infection in a non-human mammal that has been exposed to the infectious agent designates at least a reduction in the development of the infectious agent and/or of any disease or symptom or consequence of the infectious agent.

### Boosting natural immunity

Within the context of this invention, "boosting the natural immunity" refers to any improvement or stimulation of the immune system of a non-human mammal, typically in order to improve resistance or defense of said mammal against diseases.

Preferably, the term "boosting the natural immunity" designates the ability to increase the recruitment of immune cells such as monocytes, or the differentiation to dendritic cells, or the secretion of Interleukin 8. The present invention surprisingly demonstrates that SAA-3 protein has the ability to increase the recruitment of immune cells (monocyte isolation, differentiation to dendritic cells and Interleukin 8 expression pattern) and therefore to boost the natural immunity of a non-human mammals

### Non-human mammal's welfare

Intensive milk production puts special demands on the management of the health of the udder because good udder health helps, for example, dairy farmers to save costs, achieve more in terms of milk quality, production and profitability. Only healthy non human mammals with healthy udders can produce guaranteed pathogen and residue-free milk, and ensure food safety for the consumer. Therefore, controlling the udder health can be an economically advantageous way that also contributes to decrease pain of the non-human mammals and therefore contributes to its welfare. The number and types of drugs available to farm animals for the treatment of pain and inflammation and information on their effectiveness are limited.

The present invention surprisingly demonstrates that SAA-3 protein has the ability to stimulate mammary gland involution by enhancing metalloproteinase activity, modifying the SCC as well as milk fat and protein content and therefore have a benefic effect on the udder health.

Further aspects and advantages of the invention will be disclosed in the following experimental section, which illustrates the invention.

### Examples

### Example 1: Preparation of SAA-3 protein

The EcUR206 strain (an E. coli BL21 Star (DE3)-pET101/D-TOPO vector containing the goat M-SAA3 sequence) was used in recombinant protein production. The process has been explained elsewhere (Domènech et al., 2012). Briefly, BL21/pURAD1 was grown in 400 ml of LB-Amp media at an initial OD600 of 0.05 until log phase was achieved. Recombinant expression was induced by IPTG 0.1 mM for 1 h and 20 min. Cell pellet was obtained by centrifugation at 6000g for 10 min, and frozen at -80°C until use. Cell pellets were resuspended to a OD600=100 in 20 mM Na2HPO4 /NaH2PO4,0.5 M NaCl, pH 7.4 buffer containing lysozyme (0.2 mg/ml), DNase I and RNase A (20 µg/ml), cocktail inhibitor of proteases (1 mM) and MgCl2 (1 mM) during 30 min at room temperature. The suspension was mixed with pre-weighted 0.1 mm glass beads (range 26-36 mg per ml of sample) (Biospec Product, Inc, Bartlesville, USA). Three cycles of beating of 45 sec each, with one min on ice within cycles, were carried out in the MiniBead Beater (Biospec Product, Inc, Bartlesville, USA). The disrupted cell suspension was centrifuged for 15 min at 20,000g at 4°C and the supernatant was considered the soluble fraction containing the recombinant protein. Recombinant protein was purified with commercial His Spin Trap columns (GE Healthcare, Uppsala, Sweden), following manufacturer instructions. The purified protein was dialyzed ON at 4°C against PBS and it was further quantified using spectrophotometry ((A280 × Molecular weight)/Extinction coefficient; mg/ml). Lipopolysaccharide (LPS) traces in the eluted fraction were quantified by endoLISA endotoxin assay (Hyglos). Final LPS levels corresponded to 0.08 ng LPS/µg recombinant protein. This level was similar to the traces observed in the recombinant commercial Apo-SAA (ProSci incorporated, endotoxin levels less than 0.1 ng/µg protein).

### Example 2: Study of the efficiency of SAA-3 protein on bovine welfare by measuring effect on Somatic Cell Counts (SCC), metalloproteinases activity and fat and protein content after mammary gland infusion

Two quarters of nine cows were intra-mammary infused using mammary cannulas with 1 mg of M-SAA3 and 80 ng E. coli LPS (Sigma) (= control) to reproduce the possible effect of LPS traces in purified recombinant SAA-3 fraction. The same volume (10 ml) of saline solution was infused in respective control quarters (front or back quarters). Immediately after treatment all quarters were treated intra-mammary with routine antibiotic (Orbenin extra dry cow, Pfizer). Front and back quarters were treated independently statistically. Milk samples were taken the first day at 8 a.m before intra-mammary infusion (T=0) and every day at 8 a.m during 3 consecutive days (T=1, T=2 and T=3). Ten ml of milk were frozen for metalloproteinases analyses and fresh milk was analyzed for somatic cell count (SCC), fat and protein. Fresh milk samples were processed in ALLIC (Laboratori interprofessional lleter de Catalunya, Cabrils, Spain).

*Statistical analyses:* Data pertaining to the SCC, fat, protein, metalloproteinase and gene expression activity were analyzed mixed-effects model. Data were previously transformed when necessary. Results are expressed as the means of non-transformed data ± SEM (except otherwise stated).

### A. Metalloproteinases activity

Frozen milk was thawed and centrifuged at 2,700g for 10 min to obtain skimmed milk. Diluted 1:20 skimmed milk was mixed 1:1 with loading buffer (0.125M Tris pH=6.8 with 0.005% bromophenol blue, 20% glycerol, 4% SDS) and run in a 10% polyacrylamide gel with 1 mg/ml porcine gelatin. Samples were run for 1 hour at 150V in running buffer (192mM Gly, 25mM Tris, 0.1 % SDS). After running, gel was washed with 2.5% Triton X-100 solution at room temperature (RT) with gentle agitation for 30 min. Then, the gel was incubated with developing buffer (50mM Tris pH 7.6 with 0.2M NaCl, 5mM CaCl2, 0.02% Brij) during 30 min, and reincubated in fresh developing buffer for 48 hours at 37°C without agitation. Gels were stained with Comassie Blue R-250 (Bio-Rad) during 30 min. Destaining buffer (50% methanol, 10% acetic acid) was used to visualize the bands corresponding to the metalloproteinases. Gels were digitalized and band size and intensity was analyzed with the Quantity One software.

Milk metalloproteinases activity was evaluated through zymography gel quantification. The MMP-9 and MMP-2 enzymes were observed as clear bands at 92 KDa and 72 KDa, respectively. A significant M-SAA3 treatment effect was observed (P < 0.0001) (Fig 2). The M-SAA3 increased (P < 0.0001) the activity of MMP-9 during the three days of the experiment compared to the saline solution. The M-SAA3 also increased (P < 0.001) the levels of MMP-9 activity compared to LPS (Fig. 2a). In contrast, there were no differences between LPS and their negative control. On the other hand, the MMP-2 activity remained unaltered after the treatment either with M-SAA3 or LPS (Fig 2b).

The M-SAA3 clearly increased the MMP-9 activity on milk during the early dry off. The activity levels of MMP-9 (Fig 2a) were greater after 24 hours post infusion and remained beyond the basal levels during all the experiment. The LPS or endotoxin, is the major constituent of the Gram negative cell wall, and highly activates immune response. During the M-SAA3 production, residual LPS traces were detected in the final eluted protein. Thus, the LPS traces had to be evaluated, as the bovine mammary gland is highly sensitive to low doses of LPS. The traces of LPS did not increase the MMP-9 activity, observing similar levels to its respective negative control. On the other hand, MMP-2 activity remained unaltered during all the experiment (Fig 2b).

It is known that metalloproteinases have a key role during the onset of the early involution, as promote the degradation of the basement membrane of the mammary gland and therefore can be considered are key markers for mammary gland involution activity. Indeed, MMP-9 has been described as the most active metalloproteinase in the cow mammary involution process and the main source of MMP-9 is considered to be the neutrophil fraction. MMP-2 has been described to increase their levels during mammogenesis and also during the late involution, being the endothelial cells the major MMP-2 producers in bovine.

### B. Somatic Cell Counts (SCC)

A numerical increase in the SCC counts was observed in the M-SAA3 (Fig 3a) in funded quarters during the second day of the experiment.

Contrary, the effect of the LPS traces in the SCC was different. A lower numerical increase was observed only in the first day of the experiment. The somatic cell counts represent the quantification of the immune response cells that are present in milk, mainly referred as neutrophils, macrophages and other cells types such epithelial cells. As described in the scientific literature, the SCC increase normally during the involution process, as immune response mediators are attracted from blood vessels. An increase in immune response mediators would help to the protection of the mammary gland against infections, and also to increase the activity of the MMP-9.

### C. Milk fat and protein content

Fat and milk protein concentrations (Fig 3b, c) were both increased in the M-SAA3 treatment at early dry period. Indeed, a treatment effect was observed (P < 0.05) for the fat content (Fig 3b). The M-SAA3 increased (P < 0.01) fat levels compared to the respective negative control. In addition, fat content was not increased by LPS treatment compared to its negative control. A tendency (P = 0.051) in the treatment effect was observed for protein content (Fig 3c). The M-SAA3 increased (P < 0.01) the protein concentration in milk during the three days of the experiment compared to its negative control.

Protein content in M-SAA3 treated quarters tended (P = 0.06) to be greater than the LPS quarters while protein content with LPS did not differ from its negative control.

Fat and milk protein concentrations (Fig 3b, c) were both increased in the M-SAA3 treatment at early dry period. Total protein concentrations increase in early involution, partially because of water reabsorption from the secretion and partly due to increased concentrations of lactoferrin, serum albumin and immunoglobulins although expression of milk-specific proteins such as casein is decreased. Concentrations of milk fat decline slowly during the first 2-3 wk of the dry period but it is possible that in early dry period samples we still detect fat accumulation which could be higher in M-SAA3 quarters by accentuated water reabsorption.

These studies on Somatic Cell Counts (SCC), metalloproteinases activity and fat and protein content after mammary gland infusion point out the role of the M-SAA3 during the mammary gland involution.

### Example 3: Study of the efficiency of SAA-3 protein on IMIs by measuring the inhibition of S.aureus translocation and IL-8 expression in mammary gland primary cultures

### Mammary gland primary cultures

Mammary gland tissue was obtained at slaughterhouse and transported in chilled PBS with 100 µg/ml streptomycin, 100 U/ml penicillin and 2.5 µ/ml amphotericinB. In the laboratory, tissue was cut into small pieces and incubated in Hanks balanced salt solution with 0.1 mM EDTA and 0.1 mM DTT for 30 min at 37°C in 10%CO2 at 150 rpm. Then, supernatant was removed and RPMI 1640 media with 0.05 % collagenase was added and incubated for 30 min. The media contained epithelial cells, which were centrifuged at 800g for 5 min. This process was repeated 3 times. Final cell pellets were resuspended in F-12 media with 8 µg/ml bovine insuline, 10 µg/ml gentamycin, 50 µg/ml hydrocortisone, 100 µg/ml streptomycin, 100 U/ml penicillin and 2.5 µg/ml amphotericin. Cells were quantified by haemocytometer counting and incubated at 80.000 cells/cm² in flasks until differentiation. Epithelial cell phenotype was confirmed by immunofluorescence staining against anti-cytokeratin antibodies (Sigma) as previously described in literature (Hashim et al, 2004). Mammary gland primary cells were grown on coverslips and fixed with 4% paraformaldehide (diluted in PBS). SK-BR3 were used as positive control and macrophage differentiated THP-1 cells as negative control. Coverslips were blocked with 150 µl of PBS containing goat serum (0.05 % v/v) and triton 0.2%, for 30 min at room temperature (RT). Primary antibody (monoclonal anti-cytokeratin pan antibody produced in mouse, Sigma, 1:500 dilution) was incubated in blocking buffer for 2 hours at RT. After 3 washes with PBS, secondary antibody (antimouse-FITC, Sigma, 1:1000) was incubated for 1 hour at RT. Finally, cells were washed and dried coverslips, mounted into glass slides using Fluoroprep and observed in the fluorescence microscope.

### A. S.aureus translocation

### Staphylococcus aureus isolation

Pathogenic *Staphylococcus aureus* was isolated from mastitic milk and kindly provided by ALLIC (Laboratori interprofessional lleter de Catalunya, Cabrils, Spain). The S. aureus were grown in Nutrient media and plated onto mannitol salt agar. Single colonies were grown in 10 ml of Nutrient media over-night at 37°C in static conditions. Cell pellet was obtained by centrifugation at 6000g for 10 min at 4°C. Bacterial concentration was calculated by spectrophotometric quantification (DO600= 0.4 corresponds to 10E7 CFU/ml). Bacterial doses of infections were obtained by resuspending and diluting the cell pellet with the corresponding media without antibiotics.

### Staphylococcus aureus infection of mammary gland primary cultures

Mammary epithelial cells were seeded in 24-well plates at 44.0000 cells/well. Cells were preincubated with 30 ug/ml of recombinant M-SAA3 during 1 hour. After preincubation, mammary cells were infected with S. *aureus* at 10E6 CFU/ml for 2 hours. Cells were washed thoroughly with Phosphate Buffer Saline (PBS) and internalized S. *aureus* was released from cells using Triton 0.1%. Bacteria were serially diluted and plated onto mannitol salt agar. Colonies were counted after ON growth at 37°C.

The epithelial origin of the cultured cells was demonstrated by immunofluorescence staining with a monoclonal anti-pan cytokeratin (Sigma) (Figure 4 a-c). Mammary gland primary cultures preincubated with or without M-SAA3 were challenged with a pathogenic strain of S. *aureus* isolated from mastitic milk. The M-SAA3 decreased (P < 0.05) the bacterial translocation up to 25% (data represented as CFU/ml) (Fig 4e).

M-SAA3 decreased in 25% the bacterial infection of primary mammary gland cultures challenged with S. *aureus,* a pathogenic strain of isolated from mastitic milk.

IMIs and especially mastitis is the main cause of economical losses in dairy farms, over $2 billion per year in United States. In addition, S. *aureus* infection is one of the major producing mastitis, and one of the most difficult to control by antibiotic treatment.

### B. IL-8 expression

The effect of M-SAA3 on IL-8 expression as a main cytokine recruiting neutrophils was evaluated on epithelial primary cultures from cow mammary gland. The epithelial origin of the cultured cells was demonstrated by immunofluorescence staining with a monoclonal anti-pan cytokeratin (Sigma) (Figure 4 a-c). A 15-fold increased (P < 0.0001) expression of IL-8 was observed after 3 hours incubation with the M-SAA3 (Fig 4d).

The numerical SCC increase during the day 2 of the experiment (Fig 3a) suggested that M-SAA3 could be promoting cell chemotaxis. This is in agreement with previous data that indicated that hepatic SAA forms activated migration of monocytes and polimorphonuclear cells to the site of inflammation. This chemotactic attraction could be mediated by the IL-8, whose main function is related to the attraction of immune response mediators. To corroborate this hypothesis, primary cultures from bovine mammary gland tissue were obtained and incubated with the SSA-3 protein. Mammary epithelial cells increased dramatically the expression of IL-8 indicating that the mammary gland epithelial cells were able to release a chemotactic cytokine after M-SAA3 exposure. Other cell types such monocytes and neutrophils have been also described in scientific literature to produce IL-8 in response to members of the SAA family.

These studies on of *S.aureus* translocation and IL-8 expression in mammary gland primary cultures point out a key role of the M-SAA3 to control IMIs and especially mastitis.

### Example 4: Study of the efficiency of SAA-3 protein on immune response boost by measuring activation of IL-8 expression and Dendritic Cells (DC) maturation

### Bovine Dendritic Cells isolation

Bovine blood with sodium heparin 50 UI/ml, as anitcoagulant was obtained at slaughterhouse. The blood was diluted 1:1 with PBS-2% Fetal Calf Serum (FCS) at room temperature (RT). The diluted blood was layered onto Histopaque-1077 (Sigma) at 1:2 ratio (histopaque/diluted blood) and centrifuged at 600g (brakes off) during 30 minutes at RT. Peripheral blood mononuclear cells (PBMCs) were isolated from the interphase, washed with media and centrifuged 10 minutes at 200g. The cell pellet was resuspended and incubated with 10 ml of Red Blood Lysis buffer (Sigma) and incubated for 10 minutes at RT. After centrifuging at 250 g for 5 min, the process was repeated until a free-erythrocyte pellet was observed. PBMCs were resuspended in supplemented media (RPMI-10% FCS, 50 µM β-mercapthoethanol, 1% penicillin/streptomycin) and quantified using a haemocytometer. PBMCs cells were incubated in a 75 cm2 Falcon in a ratio of 10E6 cells/cm2, and incubated during 1.5 hours in 5% CO2 at 37°C. After incubation, adherent monocytes were washed once with PBS and incubated with supplemented media with Interleukin 4 (IL-4) and granulocyte macrophage colony-stimulating factor (GM-CSF) cytokines (Bovine Dendritic Cell Growht kit, Bionova, 1:20 dilution). Media was changed at day 3 and total cell differentiation by microscopy was observed at day 6. The day of the experiment, cells were scrapped and quantified. An amount of 4·105 dendritic cells/well wee seeded in 24-well plates, and incubated with supplemented RPMI without antibiotics.

### M-SAA3 treatment on bovine Dendritic Cells

Mammary epithelial cells from primary cultures (44.000 cells/well) and dendritic cells (4·10E5 cells/well) were seeded in 24-well plates. Cells were incubated with the corresponding media without antibiotics, with and without 30 µg/ml of recombinant M-SAA3 during 3 hours. After washing with PBS, cells were resuspended with 0.5 ml of Trizol (invitrogen) and frozen at -80°C until gene expression analyses by quantitative RT-PCR were performed.

### Quantitative RT-PCR

Total RNA was extracted from epithelial mammary cells and dendritic cells using Trizol (Invitrogen) following manufacturer's instructions. One microgram of RNA was retrotranscribed to cDNA using IScript cDNA synthesis kit (Bio-Rad, California, USA). Quantitative RT-PCR was performed using specific primers (Table 1). Each set of primers were individually optimized. The specificity of the amplification was evaluated by the single band identification at the expected molecular weight and a single pick in melting curves. The efficiency was calculated by amplifying serial 1/10 dilutions of each gene amplicon. A standard curve of Ct versus log concentration was plotted to obtain the efficiency, which is calculated using the formula 10^{1/slope}, with an acceptable range of 1.8-2.2. A total reaction volume of 20 µl was used, containing 50 ng of cDNA, 10 µl of SYBER Green Fluorescent (Bio-Rad), and the optimized primer concentration for each gene (Table 1). The qPCR reactions were cycled as follows: an initial denaturing step of 10 min at 95°C, followed by 40 cycles of 10s at 95°C, 15s at optimized annealing temperature for each gene, 30 s at 72°C and a final extension of 10 min at 72°C. Relative gene expression was calculated using the 2^{ΔCt} method with ACTB as reference gene control.

Dendritic Cells were incubated with or without M-SAA3. A set of genes related to activation and maturation were evaluated (Fig 5 a-f).

The IL-8 levels increased (P < 0.0001) 2-fold after treatment with M-SAA3. The INFγ was highly upregulated (P < 0.0001), obtaining levels 28-fold greater after treatment with M-SAA3. The TNFα levels were increased (P < 0.0001) in 7-fold after treatment with M-SAA3. The CCR7 marker increased (P < 0.01) 2.5-fold (after treatment with M-SAA3. The CD80 marker tended (P = 0.053) to increased 3- after treatment with M-SAA3. Finally, iNOS expression tended (P = 0.054) to increase 3-fold after treatment with M-SAA3.The Dendritic Cells (DC) are professional antigen presenting cells, with a key role in the onset of the adaptative immune response. The DC may be the only cell type able to activate naïve T cells. Circulating immature DC in the blood stream enter to tissues, where become resident cells. In mammary gland, DC populations have been identified among alveoli, epithelia and interalveolar tissue. Immature DC presents a high phagocytic activity and are responsible for antigen uptake. Maturation is reached, among other stimulus, by bacterial recognition. Mature DC reduces slightly the phagocytic activity and enhance other features such antigen presentation and the capacity to migrate towards secondary lymphatic nodes. Maturation can be detected by an increase of related molecules such as CD80 and CCR7. The CD80 is an important co-stimulatory molecule that participates in the antigen presentation along with the major histocompatibility complex II (MHCII). The CCR7 is a receptor that helps the migration of mature DC. The infused M-SAA3 increased several maturation markers related to DC activation, migration and antigen presentation (Fig 5a-f).

Pro inflammatory cytokines such as IL-8, INFγ and TNFα were upregulated. The CCR7 and CD80 markers were also increased. Finally, the expression of the enzyme oxid nitric synthase (iNOS) was also raised under M-SAA3 treatment.

These results illustrates that SAA-3 treatment increased Nitric Oxide in macrophages, a mediator that participates in the fight against pathogens and it is produced by iNOS.

These studies on DC maturation point out a key role of the M-SAA3 in a mediator that participates in the fight against pathogens by enhancing an immune response boost.

**TABLE 1**

| **Gene** | **Fw** | **Rv** | **At** | **µM** | **Amplicon** |
|---|---|---|---|---|---|
| **ACTB** | CTGGACTTCGAGCAGGAGAT | CCCGTCAGGAAGCTCGTAG | 57 | 0.125 | 75 |
| **IL-8** | TTGAGAGTGGGCCACACTGTG | TGCACCCACTTTTCCTTGG | 55 | 0.5 | 100 |
| **INFγ** | ATAACCAGGTCATTCAAGG | ATTCTGACTTCTCTTCCGCT | 50 | 0.5 | 218 |
| **CCR7** | AGCACGTGGAGGCCTTGAT | GCGGATGATGACGAGGTAGC | 50 | 0.5 | 100 |
| **CD80** | GAACCGCACCATCACTGACA | TAATGGTCCAGGTCAGGTGC | 56 | 0.25 | 484 |
| **IL-12** | GAGGCCTGTTTACCACTGGA | CTCATAGATACTTCTAAGGCACAG | 50 | 0.3125 | 140 |
| **TNFα** | AACAGCCCTCTGGTTCAAAC | TCTTGATGGCAGACAGGATG | 60 | 0.5 | 296 |
| **iNOS** | CACAACGGCAACATCAGG | TAAGCAGGACTAGAGGCAACA | 60 | 0.5 | 226 |

| | | | | | |
|---|---|---|---|---|---|
| **Table 1** Sequence, annealing temperature (At), concentration (µM) and amplicon (bp) of the primers used for qPCR. | | | | | |

### Sequences

**SEQ ID NO: 1: M-SAA3 mRNA sequence**

CACCAGGAGCCTCAGCAGGAGGGCACGGCCACAGGATGAACCTTTCCACGGCATCATTTTCTGCTTCCT
GATCCTGGGCGTCAGCAGCCAGAGATGGGGGACATTCCTCAAGGAAGCTGGTCAAGGGGCTAAAGACATG
TGGAGAGCTTACCAAGACATGAAAGAAGCCAACTACAGGGGTGCAGACAAATACTTCCACGCCCGTGGAA
ACTATGACGCTGCCCGAAGGGGACCTGGGGGTGCCTGGGCTGCTAAAGTGATCAGTAACGCCAGAGAGAC
TATTCAGGGAATCACAGACCCTCTGTTTAAGGGTATGACCAGGGACCAGGTACGGGAGGATTCGAAGGCC
GACCAGTTTGCCAACGAATGGGGCCGGAGCGGCAAAGACCCCAACCACTTCAGACCTGCTGGCCTGCCTG
ACAAGTACTGAGCTGCCTCTCTCTCTGCTCAGGAGATGGGCTGTGAGTCCCTAAGGGCAGAGACACTGAC
CTAGAGAGTTCTCTGTCCTCAGAAGGCAGCAGATCTAATAAATGCTCAAGAGATGG

**SEQ ID NO: 2: M-SSA3 protein sequence**

MNLSTGIIFCFLILGVSSQRWGTFLKEAGQGAKDMWRAYQDMKEANYRGADKYFHARGNYDAARRGPGGA
WAAKVISNARETIQGITDPLFKGMTRDQVREDSKADQFANEWGRSGKDPNHFRPAGLPDKY

## Claims

1. An acute phase protein, or a composition comprising an acute phase protein, for use to control an intra-mammary infection in a non-human mammal.

2. The protein or composition of claim 1, for use to control mastitis.

3. The protein or composition of claim 1 or 2, for use to control intra-mammary S. *aureus* infection.

4. An acute phase protein, or a composition comprising an acute phase protein, for use to enhance the welfare of a non-human mammal by improving mammary gland involution.

5. The protein or composition of claim 4, for use to enhance udder health.

6. The protein or composition of claim 4, for use to improve udder inflammation.

7. An acute phase protein, or a composition comprising an acute phase protein, for use to stimulate the natural immunity in a non-human mammal.

8. A protein or composition for use of anyone of claims 1 to 7, wherein the acute phase protein is a serum amyloid A protein.

9. A protein or composition for use of claim 8, wherein the serum amyloid A protein is a SAA-3 protein.

10. The protein or composition for use of claim 9, wherein said SAA-3 protein comprises an amino acid sequence of SEQ ID NO: 2, or a fragment thereof having at least 20 consecutive amino acids of SEQ ID NO: 2; or a sequence having at least 80% identity to SEQ ID NO: 2, preferably at least 85%, 90%, 95% or 97% sequence identity to SEQ ID NO: 2.

11. The protein or composition for use of anyone of claims 1 to 10, wherein the acute phase protein is administered by intra-mammary infusion.

12. The protein or composition for use of anyone of claims 1 to 11, wherein the acute phase protein is administered during the dry off period.

13. The protein or composition for use of anyone of the preceding claims, which further comprises administering to the non-human mammal at least one additional active agent, preferably selected from antibiotics, anti-oxidants, antiprolactins, antigens and/or tip sealeants.

14. The protein or composition for use of claim 13, wherein the at least one additional agent is selected from benzylpenicillin, cloxacillin, nafcillin, cephems, aminoglycosides, gentamicin, kanamycin, dihydrostreptomycin, erythromycin, tylosin, oxytetracycline, benzylpenicillin, dihydrostreptomycin, methicillin, lincomycin, clindamycin, chloramphenicol, florfenicol, virginiamycin, tilmicosin, and nisin.

15. The protein or composition for use of any one of the preceding claims, wherein the non-human mammal is a ruminant, preferably a bovine, more preferably a cow.

16. A veterinary composition comprising a SAA protein and an acceptable excipient.

17. A composition of claim 16, said composition being in the form of a viscous paste.

18. A composition of claims 16 and 17, wherein the SAA protein is a SAA-3 protein.

19. A composition comprising (i) a SAA protein and (ii) an antibiotic, an antioxidant, an antiprolactin and/or an antigen compound with optionally a tip sealant, for combined or separate formulation.

20. A composition comprising (i) a SAA protein and (ii) a tip sealant with optionally an antibiotic, an antioxidant, an antiprolactin and/or an antigen compound, for combined or separate formulation.


SEQUENCE LISTING

<110>  CEVA SANTE ANIMALE

<120>  Compositions and Method for controlling infections in non-human
       mammals using acute phase proteins

<130>  B1621EP00

<160>  18

<170>  PatentIn version 3.3

<210>  1
<211>  546
<212>  DNA
<213>  bovine SAA-3

<400>  1
caccaggagc ctcagcagga gggcacggcc acaggatgaa cctttccacg ggcatcattt      60

tctgcttcct gatcctgggc gtcagcagcc agagatgggg gacattcctc aaggaagctg     120

gtcaaggggc taaagacatg tggagagctt accaagacat gaaagaagcc aactacaggg     180

gtgcagacaa atacttccac gcccgtggaa actatgacgc tgcccgaagg ggacctgggg     240

gtgcctgggc tgctaaagtg atcagtaacg ccagagagac tattcaggga atcacagacc     300

ctctgtttaa gggtatgacc agggaccagg tacgggagga ttcgaaggcc gaccagtttg     360

ccaacgaatg gggccggagc ggcaaagacc ccaaccactt cagacctgct ggcctgcctg     420

acaagtactg agctgcctct ctctctgctc aggagatggg ctgtgagtcc ctaagggcag     480

agacactgac ctagagagtt ctctgtcctc agaaggcagc agatctaata aatgctcaag     540

agatgg                                                                546

<210>  2
<211>  131
<212>  PRT
<213>  bovine SAA-3

<400>  2

Met Asn Leu Ser Thr Gly Ile Ile Phe Cys Phe Leu Ile Leu Gly Val
1               5                   10                  15

Ser Ser Gln Arg Trp Gly Thr Phe Leu Lys Glu Ala Gly Gln Gly Ala
            20                  25                  30

Lys Asp Met Trp Arg Ala Tyr Gln Asp Met Lys Glu Ala Asn Tyr Arg
        35                  40                  45

Gly Ala Asp Lys Tyr Phe His Ala Arg Gly Asn Tyr Asp Ala Ala Arg
    50                  55                  60

Arg Gly Pro Gly Gly Ala Trp Ala Ala Lys Val Ile Ser Asn Ala Arg
65                  70                  75                  80

Glu Thr Ile Gln Gly Ile Thr Asp Pro Leu Phe Lys Gly Met Thr Arg
                85                  90                  95

Asp Gln Val Arg Glu Asp Ser Lys Ala Asp Gln Phe Ala Asn Glu Trp
            100                 105                 110

Gly Arg Ser Gly Lys Asp Pro Asn His Phe Arg Pro Ala Gly Leu Pro
        115                 120                 125

Asp Lys Tyr
    130

<210>  3
<211>  20
<212>  DNA
<213>  Artificial

<220>
<223>  primer

<400>  3
ctggacttcg agcaggagat                                                   20

<210>  4
<211>  19
<212>  DNA
<213>  Artificial

<220>
<223>  primer

<400>  4
cccgtcagga agctcgtag                                                    19

<210>  5
<211>  21
<212>  DNA
<213>  Artificial

<220>
<223>  primer

<400>  5
ttgagagtgg gccacactgt g                                                 21

<210>  6
<211>  19
<212>  DNA
<213>  Artificial

<220>
<223>  primer

<400>  6
tgcacccact tttccttgg                                                    19


<210>  7
<211>  19
<212>  DNA
<213>  Artificial

<220>
<223>  primer

<400>  7
ataaccaggt cattcaagg                                                    19


<210>  8
<211>  20
<212>  DNA
<213>  Artificial

<220>
<223>  primer

<400>  8
attctgactt ctcttccgct                                                   20


<210>  9
<211>  19
<212>  DNA
<213>  Artificial

<220>
<223>  primer

<400>  9
agcacgtgga ggccttgat                                                    19


<210>  10
<211>  20
<212>  DNA
<213>  Artificial

<220>
<223>  primer

<400>  10
gcggatgatg acgaggtagc                                                   20


<210>  11
<211>  20
<212>  DNA
<213>  Artificial

<220>
<223>  primer

<400>  11
gaaccgcacc atcactgaca                                                   20

<210>  12
<211>  20
<212>  DNA
<213>  Artificial

<220>
<223>  primer

<400>  12
taatggtcca ggtcaggtgc                                                    20


<210>  13
<211>  20
<212>  DNA
<213>  Artificial

<220>
<223>  primer

<400>  13
gaggcctgtt taccactgga                                                    20


<210>  14
<211>  24
<212>  DNA
<213>  Artificial

<220>
<223>  primer

<400>  14
ctcatagata cttctaaggc acag                                               24


<210>  15
<211>  20
<212>  DNA
<213>  Artificial

<220>
<223>  primer

<400>  15
aacagccctc tggttcaaac                                                    20


<210>  16
<211>  20
<212>  DNA
<213>  Artificial

<220>
<223>  primer

<400>  16
tcttgatggc agacaggatg                                                    20


<210>  17

<211>  18
<212>  DNA
<213>  Artificial

<220>
<223>  primer

<400>  17
cacaacggca acatcagg                                                    18


<210>  18
<211>  21
<212>  DNA
<213>  Artificial

<220>
<223>  primer

<400>  18
taagcaggac tagaggcaac a                                                21